# EUROPEAN PATENT SPECIFICATION

(11) **EP 1 852 095 B1**
(45) Date of publication and mention of the grant of the patent: **23.12.2009**
(21) Application number: 07106967.8
(22) Date of filing: 25.04.2007
(51) Int. Cl.: A61F 9/007

(54) **Irrigation/aspiration tip**
Spül- und Absaugspitze
Pointe d'irrigation/aspiration

(30) Priority: 05.05.2006 US 429568
(43) Date of publication of application: 07.11.2007
(73) Proprietor: Alcon, Inc., 6331 Hünenberg (CH)
(72) Inventor: Boukhny, Mikhail, Laguna Niguel, CA 92677 (US); Liao, Grace C., Irvine, CA 92620 (US)
(74) Representative: Hanna, Peter William Derek

(56) References cited:
- EP-A- 1 371 347
- EP-A- 1 607 077
- WO-A-98/07398
- US-B1- 6 398 759

## Description

### Background of the Invention

This invention relates generally to the field of cataract surgery and more particularly to irrigation/aspiration tips used during phacoemulsification removal surgery.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible fluid tubings. Through the electric cable, the console varies the power level transmitted by the handpiece to the attached cutting tip and the flexible fluid tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip. Once the harder lens materials are removed from the eye, the surgeon typically uses an irrigation/aspiration (I/A) handpiece to remove any remaining softer materials and generally clean out any remaining lens materials from the capsular bag and any remaining viscoelastic materials from the anterior chamber.

The preferred surgical technique is to make the incision into the anterior chamber of the eye as small as possible in order to reduce the risk of induced astigmatism. These small incisions result in very tight wounds that squeeze the irrigating sleeve during use. Although reducing the diameter of the I/A tip itself is desirable, reducing the diameter of the external silicone rubber sleeve is more problematic because that same sleeve is used on the phacoemulsification tip as well. A reduced diameter sleeve will not fit over the phacoemulsification tip. Providing the surgeon with sleeves of differing diameters (one large for the phaco tip and one small of the I/A tip) can be problematic by leading to confusion over which sleeve goes with which tip.

Using a sleeve of large enough diameter to work well with a phaco tip can be oversized if used on a smaller diameter I/A/ tip. Such over sizing results in the leading edge of the sleeve getting caught on the incision during insertion into the wound. Additionally, the loose fit between the sleeve and the I/A tip allows too much of the irrigating stream to flow straight out of the distal end of the sleeve, possibly repelling the very tissue sought to be aspirated.

Therefore, a need continues to exist for a reduced diameter I/A tip that can be used with standard diameter infusion sleeves.

Irrigation/aspiration tips for use in phacoemulsification procedures, having an elastomeric infusion sleeve around an inner aspiration tube are described in WO 98/07398 (Oversby Pty. Ltd.), EP-A-1,371,347 (Oversby Pty. Ltd.), and EP-A-1,607,077 (Akahoshi, et al).

### Brief Summary of the Invention

The present invention improves upon the prior art by providing an irrigation/aspiration tip having a shaft with a reduced or relatively small diameter with a distal tip having a relatively large diameter, in accordance with claims which follow. The diameter of the shaft can vary depending upon the incision size preferred by the surgeon, and the diameter of the distal tip will vary depending upon the size of the phacoemulsification tip preferred by the surgeon.

Accordingly, one objective of the present invention is to provide a surgical 10 irrigation system having reduced irrigation flow resistance.

Another objective of the present invention is to provide a surgical irrigation system having more stable intraocular pressures.

Another objective of the present invention is to provide a surgical irrigation system that allows for higher aspiration vacuum.

Another objective of the present invention is to provide a surgical irrigation system that allows for higher aspiration flow. These and other advantages and objectives of the present invention will become apparent from the detailed description and claims that follow.

### Brief Description of the Drawings

FIG. 1 is a perspective view of the I/A handpiece of the present invention and a control console that may be used with the present invention.
FIG. 2 is an enlarged cross-sectional view of the distal end of a prior art I/A tip.
FIG. 3 is an enlarged cross-sectional view of the distal end of the I/A handpiece of the present invention.

### Detailed Description of the Invention

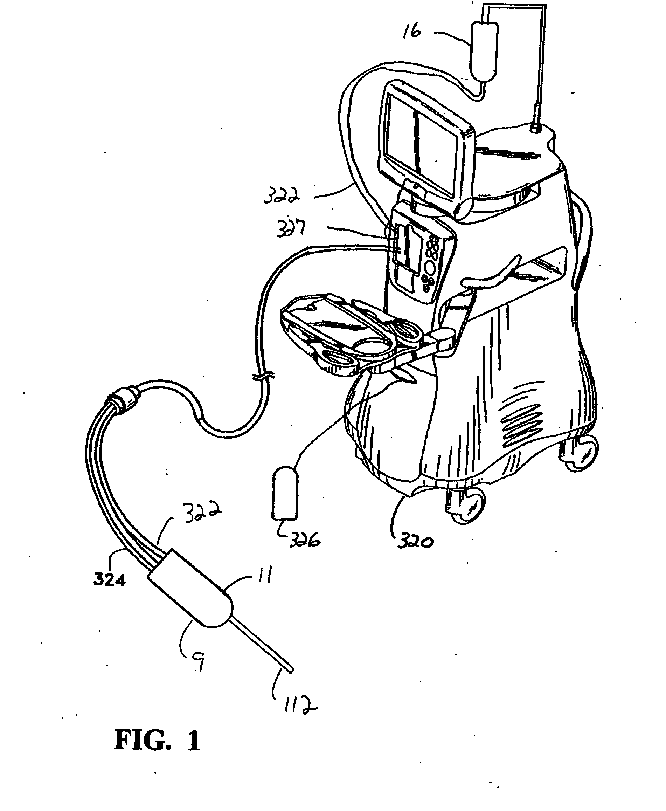

As best seen in FIG. 1, surgical console 320 suitable for use with the present invention may be any commercially available surgical control console such as the INFINITI® vision system available from Alcon Laboratories, Inc., Fort Worth, Texas. Console 320 is connected to I/A handpiece 9, which is supplied with irrigating fluid through tubings 322 from source 16, and aspiration line 324, and the flow through lines 322 and 324 and cassette 327 is controlled by the user, for example, via footswitch 326. Handpiece 9 consists of body 11 and tip 112.

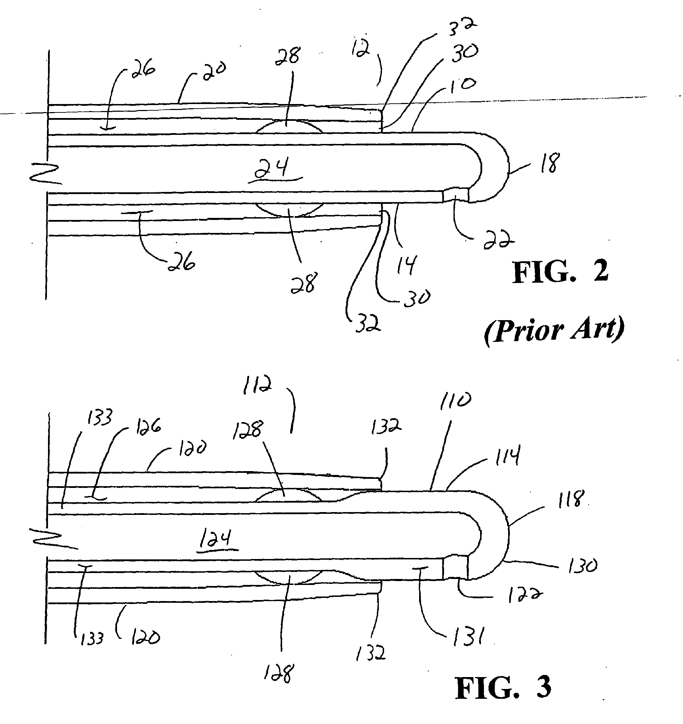

As best seen in FIG. 2, distal (or working) end 10 of prior art I/A tip 12 generally include aspirating tube 14 having closed distal end 18 coaxially mounted within hollow, tubular infusion sleeve 20. Tube 14 generally is made from stainless steel and sleeve 20 generally is made from molded silicone rubber, but other materials may also be used. Aspirating port 22 provides fluid communication between the surgical site and hollow interior 24 of tube 14. Infusion sleeve 20 forms annular infusion fluid passageway 26 between sleeve 20 and tube 14. Ports 28 cut into the sides of sleeve 20 allow infusion fluid to stream out of tip 12 at a direction perpendicular to the longitudinal axis of tip 12. As shown in FIG. 2, when the diameter of tube 14 is reduced, the inner diameter of annular gap 26 also increases, allowing for larger gap 30 to occur at distal end 32 of sleeve 20. Such a large gap 30 allows infusion fluid to stream out of gap 30 rather than through ports 28, and can repel material sought to be aspirated through port 22. Gap 30 also allows distal end 32 of sleeve 20 to be unsupported, and easily collapsed and distorted, making it difficult to insert end 10 of tip 12 into a tight wound.

As best seen in FIG. 3, distal (or working) end 110 of I/A tip 112 of the present invention generally include aspirating tube 114 having closed distal end 118 coaxially mounted within hollow, tubular infusion sleeve 120. Tube 114 generally is made from stainless steel and sleeve 120 generally is made from molded silicone rubber, but other materials may also be used. Aspirating port 122 provides fluid communication between the surgical site and hollow interior 124 of tube 114. Infusion sleeve 120 forms annular infusion fluid passageway 126 between sleeve 120 and tube 114. Ports 128 cut into the sides of sleeve 120 allow infusion fluid to stream out of tip 112 at a direction perpendicular to the longitudinal axis of tip 112. Distal end 130 of tube 114 contains an enlarged portion 131 that has an outer diameter that is larger than shaft 133 of tube 114 and that approximates the inner diameter of sleeve 120, providing a relatively tight fit between sleeve 120 and distal end 130 of tip 112. Such a construction virtually eliminates gap 30, thereby making tip 112 easier to insert into a tight wound and forces more infusion fluid out of ports 128.

This description is given for purposes of illustration and explanation. It will be apparent to those skilled in the relevant art that changes and modifications may be made to the invention described above without departing from its scope. For example, the figures illustrate tip 112 being straight, but one skilled in the art will recognize that tip 112 may be formed in any suitable shape, such as angled or bent.

## Claims

1. An irrigation/aspiration tip (112) adapted to be mounted to a surgical handpiece body (11), comprising;
an aspirating tube (114) comprising a shaft portion (133) and a distal end portion (130) containing an enlarged portion (131) that has an outer diameter that is larger than that of the shaft portion (133);
an elastomeric infusion sleeve (120) coaxially mounted around the aspirating tube, so as to form an annular fluid passageway (126) between the sleeve and the tube;
**characterized in that** the outer diameter of the enlarged portion (131) of the distal end portion (130) of the aspirating tube (114) approximates to that of the internal diameter of the infusion sleeve (120), wherein the distal end portion (130) provides a relatively tight fit between the sleeve and the distal end portion, so as to virtually eliminate a gap (30) between the end of the sleeve and the aspirating tube,
and **in that** the elastomeric infusion sleeve (120) comprises ports (128) formed in the sides of the sleeve to allow infusion fluid to stream out of the tip at a direction perpendicular to the longitudinal axis of the tip.

2. The irrigation/aspiration tip of claim 1, wherein the infusion sleeve (120) comprises a hollow tube with an internal diameter and the shaft portion (133) has a reduced diameter relative to the internal diameter of the sleeve.

3. The irrigation/aspiration tip of claim 1 or claim 2, wherein the distal end portion (130) comprises a closed distal end (118) and an aspirating port (122).

4. The irrigation/aspiration tip of claim 1, wherein the elastomeric infusion sleeve (120) is made of silicone rubber.

5. The irrigation/aspiration tip of claim 1, wherein the aspirating tube (114) is made of stainless steel.

## Patentansprüche

1. Spül-/Absaugspitze (112), die zum Anbringen an einem chirurgischen Handstückkörper (11) eingerichtet ist, mit:
einem Absaugrohr (114), das einen Schaftabschnitt (133) und einen distalen Endabschnitt (130) mit einem erweiterten Abschnitt (131) aufweist, dessen Außendurchmesser größer ist als der des Schaftabschnitts (133);
einem Elastomer-Infusionsmantelschlauch (120), der koaxial um das Absaugrohr angeordnet ist, um einen ringförmigen Fluidkanal (126) zwischen dem Mantelschlauch und dem Rohr zu bilden;
**dadurch gekennzeichnet, dass** der Außendurchmesser des erweiterten Abschnitts (131) des distalen Endabschnitts (130) des Ansaugrohrs (114) näherungsweise dem Innendurchmesser des Infusions-Mantelschlauchs (120) entspricht, wodurch der distale Endabschnitt (130) einen relativ engen Sitz zwischen dem Mantelschlauch und dem distalen Endabschnitt bereitstellt, damit ein Spalt (30) zwischen dem Ende des Mantelschlauchs und dem Absaugrohr praktisch beseitigt wird,
und **dadurch**, dass der Elastomer-Infusionsmantelschlauch (120) in den Seiten des Mantelschlauchs ausgebildete Öffnungen (128) aufweist, damit Infusionsfluid senkrecht zur Längsachse der Spitze aus der Spitze herausfließen kann.

2. Spül-/Absaugspitze nach Anspruch 1, bei der der Infusionsmantelschlauch (120) einen hohlen Schlauch mit einem Innendurchmesser aufweist und der Schaftabschnitt (133) einen verringerten Durchmesser relativ zum Innendurchmesser des Mantelschlauchs hat.

3. Spül-/Absaugspitze nach Anspruch 1 oder 2, bei der der distale Endabschnitt (130) ein geschlossenes distales Ende (118) und eine Absaugöffnung (122) aufweist.

4. Spül-/Absaugspitze nach Anspruch 1, bei der der Elastomer-Infusionsmantelschlauch (120) aus Silikonkautschuk besteht.

5. Spül-/Absaugspitze nach Anspruch 1, bei der das Absaugrohr (114) aus Edelstahl besteht.

## Revendications

1. Pointe d'irrigation/aspiration (112) adaptée pour être montée sur un corps de pièce à main chirurgicale (11), comprenant :
un tube d'aspiration (114) comprenant une partie formant tige (133) et une partie formant extrémité distale (130) contenant une partie élargie (131) ayant un diamètre extérieur qui est supérieur à celui de la partie formant tige (133) ;
un manchon de perfusion élastomère (120) monté de manière coaxiale autour du tube d'aspiration, afin de former un passage de fluide (126) annulaire entre le manchon et le tube ;
**caractérisée en ce que** le diamètre extérieur de la partie élargie (131) de la partie formant extrémité distale (130) du tube d'aspiration (114) se rapproche de celui du diamètre interne du manchon de perfusion (120), la partie formant extrémité distale (130) fournissant un ajustement relativement serré entre le manchon et la partie formant extrémité distale, afin d'éliminer virtuellement un espacement (30) entre l'extrémité du manchon et le tube d'aspiration,
et **en ce que** le manchon de perfusion élastomère (120) comprend des orifices (128) formés sur les côtés du manchon pour autoriser le fluide de perfusion à s'écouler hors de la pointe dans une direction perpendiculaire à l'axe longitudinal de la pointe.

2. Pointe d'irrigation/aspiration selon la revendication 1, dans laquelle le manchon de perfusion (120) comprend un tube creux avec un diamètre interne et la partie formant tige (133) a un diamètre réduit par rapport au diamètre interne du manchon.

3. Pointe d'irrigation/aspiration selon la revendication 1 ou 2, dans laquelle la partie formant extrémité distale (130) comprend une extrémité distale fermée (118) et un orifice d'aspiration (122).

4. Pointe d'irrigation/aspiration selon la revendication 1, dans laquelle le manchon de perfusion élastomère (120) est réalisé en caoutchouc de silicone.

5. Pointe d'irrigation/aspiration selon la revendication 1, dans laquelle le tube d'aspiration (114) est réalisé en acier inoxydable.
